# EUROPÄISCHE PATENTANMELDUNG

(11) **EP 2 292 235 A2**
(43) Veröffentlichungstag der Anmeldung: **09.03.2011**
(21) Anmeldenummer: 10194145.8
(22) Anmeldetag: 20.07.2007
(51) Int. Cl.: A61K 31/519, A61P 37/00, A61P 37/06, A61K 9/00, A61P 11/06, A61P 17/06, A61P 19/00, A61P 19/02, A61P 25/28

(54) **Methotrexat-Lösungen**

(30) Priorität: 21.07.2006 DE 102006033837
(62) Teilanmeldung aus: 07786239.9
(71) Anmelder: medac Gesellschaft für klinische Spezialpräparate mbH, 22880 Wedel (DE)
(72) Erfinder: Will, Heiner, 22559 Hamburg (DE)
(74) Vertreter: Vossius & Partner

(57) **Zusammenfassung**

Es werden konzentriert Methotrexat-Lösungen beschrieben, die sich zur Verwendung eines Wirkstoffs zur Herstellung eines parenteralen zu verabreichenden Medikaments zur Behandlung von entzündlichen Autoimmunerkrankungen eignen. Das Methorexat wird dabei in einer Konzentration von größer als 25mg/ml in einem pharmazeutisch verträglichen Lösungsmittel eingesetzt. Die Erfindung betrifft auch eine Fertigspritze sowie eine Karpule, die eine derartige pharmazeutisch Lösungsformulierung enthalten, sowie einen Pen-Injektor, der eine solche Karpule und/oder eine Fertigspritze umfasst.

## Beschreibung

Die vorliegende Erfindung betrifft, konzentrierte Methotrexat-Lösungen. Insbesondere betrifft die vorliegende Erfindung die Verwendung von Methotrexat zur Herstellung eines parenteral zu verabreichenden Medikaments zur Behandlung von entzündlichen Autoimmunerkrankungen, wobei das Methotrexat in einer Konzentration von größer als 25mg/ml in einem pharmazeutisch verträglichen Lösungsmittel vorliegt. Ferner betrifft die vorliegende Erfindung eine Fertigspritze und eine Karpule, welche eine derartige pharmazeutische Lösungsformulierung enthalten, sowie einen Pen-Injelctor, der eine solche Karpule und/oder eine Fertigspritze umfasst.

Der pharmazeutische Wirkstoff N-{4-[(2,4-Diamino-6-pteridinylmethyl)methylamino]-benzovl}-L-glutaminsäure (Freiname: Methotrexat, kurz: MTX) ist seit den frühen 50iger Jahren bekannt. Methotrexat ist ein Folsäure-Antagonist. Es bewirkt als Antimetabolit der Nukleinsäuresynthese eine intrazelluläre Hemmung der Dehydrofolat-Reduktase (irreversible Bindung) mit konsekutive Inhibition der Purinsynthese, hemmt LTB₄-Synthese in Neutrophilen, hemmt IL-1-Synthese, supprimiert zellvermittelte Immunität und hemmt Endothelzellproliferation.

Lange Zeit wurde Methotrexat aufgrund seiner Wirkung als Zytostatikum bevorzugt in der Onkologie eingesetzt. Hier wurde es insbesondere für das Mammakarzinom verwendet, allerdings auch bei Kindern für die Behandlung der Leukämie. Für letztere Indikation ist Methotrexat auch heute noch von entscheidender Bedeutung. Frühzeitig wurde auch die Wirksamkeit von Methotrexat bei der Psoriasis bemerkt Da die Psoriasis auch mit einer rheumatoiden Arthritis einhergehen kann, wurde auch dies als Therapieoption Ende der 50iger Jahren erstmals in Einzelfällen beobachtet.

Die rheumatoide Arthritis wird therapeutisch in der Regel zunächst mit schnell schmerzlindernden und kurzfristig entzündungshemmenden Substanzen behandelt. Hierfür sind nicht-steroidale Antirheumatika (NSAR, z.B. der Wirkstoff Diclofenac) und Kortikoide zu nennen. Jedoch beeinflussen diese den eigentlichen Krankheitsverlauf nicht. Bei den meisten Patienten kommen NSAR und Kortikoide nur so lange zum Einsatz, bis die Entzündungen und Schmerzen deutlich abklingen. Anschließend wird oft deren Dosis reduziert oder das Präparat ganz ausgeschlichen.

Einen krankheitsmodinzierenden Effekt bei der rheumatoiden Arthritis haben nur die Disease Modifying Anti Rheumatic Drugs (DMARD's). Als Beispiele für diese auch als "Basistherapeutika" bezeichneten Wirkstoffe können neben Methotrexat auch Azathioprin, Sulfasalazin und Antimalariamittel genannt werden. Basistherapeuiika greifen direkt in das Krankheitsgeschehen ein und können den Krankheitsprozess bremsen, weshalb ein möglichst frühzeitiger Einsatz anzustreben ist. Da es sich bei der rheumatoiden Arthritis um eine chronische Krankheit handelt, sind die Basistherapeutika meist über entsprechend lange Zeiträume einzunehmen, bei guter Wirksamkeit und Verträglichkeit wird die Therapie oft lebenslang fortgesetzt (kontinuierliche Langzeittherapie), wobei die Wirkstoffdosis an den Krankheitsverlauf adaptiert werden kann.

Im Gegensatz zur Chemotherapie bei Tumorerkrankungen wird Methotrexat als Basistherapeutikum zur Behandlung der rheumatoiden Arthritis um ein Vielfaches, vereinzelt bis zu 1000-fach, geringer dosiert, weshalb man auch bei der antirheumatischen Therapie von der "niedrig-dosierten Methotrexat-Therapie" spricht. In der antirheumatischen Therapie ist in Deutschland ein Dosisbereich von 5,0 bis 30,0mg pro Woche üblich, im europäischen Ausland wird bis zu 40,0mg pro Woche dosiert. Sehr wichtig ist, dass Methotrexat nur einmal pro Woche verabreicht wird.

Die Applikation von Methotrexat kann prinzipiell oral wie auch parenteral erfolgen. Allerdings wurde die mittels Tabletten zunächst für lange Zeit oral erfolgte Therapie durch parenterale Formulierungen abgelöst, da festgestellt wurde, dass Methotrexat aus Tabletten zuverlässiger resorbiert wird und somit keine ausreichende Genauigkeit bei der dosisabhängiger Therapie gewährleistet ist. Zur parenteralen Verabreichung geeignete Zytostatika werden normalerweise durch Auflösen des Wirkstoffs in einem geeigneten Lösungsmittel hergestellt, indem man für jeden Patienten individuell eine spezifische Wirkstoffmenge verwendet. Die Handhabung von Zytostatika und die Zubereitung Zytostatika-enthaltender Arzneimittel ist allerdings nicht unproblematisch und vom Gesetzgeber mit strengen Auflagen versehen. Beispielsweise dürfen Zytostatika außerhalb einer dafür eigens geschaffenen und geeigneten Abzugsanlage nicht zubereitet werden. Da Rheumatologen und Hausärzte in der Regel nicht über entsprechende Einrichtungen verfügen, ist es ihnen nicht gestattet, Methotrexat selbst zuzubereiten, wobei bereits das Aufziehen, einer Spritze aus einer Flasche (beispielsweise einer Durchstechflache, welche die Wirkstofflösung enthält) als Zubereitung angesehen wird.

Aus diesem Grund wurden Fertigspritzen entwickelt, um den Arbeitsschritt des Aufziehens zu eliminieren. Erstmals wurden entsprechende Fertigspritzen zur subkutanen Applikation für die vorliegende Anmelderin europaweit zugelassen. Mit diesen Fertigspritzen ist die Anwendung durch Arzt, medizinisches Personal oder, bei Selbstapplikation, durch den Patienten selber möglich, ohne dass es eines dazwischengeschalteten Apothekers bedarf, der über ein geeignetes Abzugsystem verfügt.

Aus dem Stand der Technik bekannt sind in Bezug auf die Therapie der rheumatoiden Arthritis Fertigspritzen zur parenteralen Verabreichung enthaltend Methotrexat-Lösungen, in welchen der Wirkstoff in einer Konzentration von bis zu 25mg/ml in einem pharmazeutisch verträglichen Lösungsmittel vorliegt (Handelsnamen: Lantarel® der Firma Wyeth, Metex® der Anmelderin), wobei die Injektionslösung Lantarel® der Konzentration 25 mg/ml (Handelsname: Lantarel® FS 25mg) nicht zur subkutanen Applikation zugelassen ist. Methotrexat hat sich mittlerweile zum "Goldstandard" in der Behandlung der rheumatoiden Arthritis entwickelt.

Wie bereits beschrieben, ist es für eine erfolgreiche Basistherapie mit Methotrexat erforderlich, dass dem Rheumapatienten über einen sehr langen Zeitraum, mitunter ein Leben lang, wöchentlich die geeignete Dosis Methotrexat verabreicht wird. Für die parenterale Verabreichung spricht die gegenüber dem oralen Applikationsweg beschriebene vorteilhaftere Bioverfügbarkeit. Ferner haben insbesondere Kinder eine gewisse Abneigung gegen die Einnahme von Tabletten. Allerdings hat sich herausgestellt, dass gerade die subkutane Verabreichung mit Schwierigkeiten verbunden ist. Bei Therapien mit den aus dem Stand der Technik bekannten Präparaten stellte sich eine ablehnende Haltung seitens der Patienten heraus. Diese beruht auf der Problematik, im wöchentlichen Zeitabstand die erforderliche relativ große Menge an Wirkstofflösung (z.B. bei entsprechender Wirkstoffdosis bis zu 3ml) unter die Haut zu bringen, was insbesondere Kindern, den wöchentlichen Arztbesuch inbegriffen, nicht leicht zu vermitteln ist.

Es besteht somit der Bedarf für pharmazeutische Formulierungen von Methotrexat, welche dem Patienten, einschließlich Kindern, möglichst einfach und schmerzfrei bei guter Bioverfügbarkeit über einen langen Zeitraum hinweg regelmäßig, insbesondere wöchentlich, verabreicht werden kann und somit zu einer hohen "Patienten-Compliance" führt. Vorteilhafterweise sollte sich der Patient selbst die pharmazeutische Formulierung verabreichen können.

Der vorliegenden Erfindung liegt somit die Aufgabe zugrunde, eine pharmazeutische Zubereitung zur Behandlung von entzündlichen Autoimmunerkrankungen, insbesondere der rheumatoiden Arthritis, bereitzustellen, welche die vorstehend beschriebenen Nachteile der aus dem Stand der Technik bekannten Präparate überwindet.

Die der vorliegenden Erfindung zugrunde liegende Aufgabe wird gelöst durch den Gegenstand der anhängenden Patentansprüche.

In einer ersten Ausführungsform stellt die Erfindung die Verwendung von Methotrexat zur Herstellung eines parenteral zu verabreichenden Medikaments zur Behandlung von entzündlichen Autoimmunerkrankungen, wobei das Methotrexat in einer Konzentration von größer als 25mg/ml in einem pharmazeutisch verträglichen Lösungsmittel vorliegt, bereit.

In einer weiteren Ausführungsform stellt die Erfindung eine Fertigspritze bereit, welche eine pharmazeutische Lösungsformulierung von Methotrexat mit einer Konzentration von größer als 25mg/ml in einem pharmazeutisch verträglichen Lösungsmittel enthält.

Ferner stellt die Erfindung in einer weiteren Ausführungsform eine Karpule bereit, welche eine pharmazeutische Lösungsformulierung von Methotrexat mit einer Konzentration von größer als 25mg/ml in einem pharmazeutisch verträglichen Lösungsmittel enthält, sowie einen Pen-Injektor, welcher eine solche Karpule umfasst.

Gemäß der vorliegenden Erfindung werden Medikamente bzw. pharmazeutische Lösungsformulierungen bereitgestellt, welche Methotrexat mit einer Konzentration, von größer als 25mg/ml in einem pharmazeutisch verträglichen Lösungsmittel umfassen. In einer bevorzugten Ausführungsform liegt das Methotrexat in dem Medikament in einer Konzentration von größer als 25mg/ml bis etwa 150mg/ml vor. Weiterhin bevorzugt sind Konzentrationsbereiche von 30mg/ml bis 100mg/ml, sowie insbesondere 40mg/ml bis 80mg/ml und ferner 50mg/ml bis 75mg/ml. In einer besonderes bevorzugten Ausführungsform liegt das Methotrexat in dem Medikament in einer Konzentration von etwa 50mg/ml in einem pharmazeutisch verträglichen Lösungsmittel vor.

Als das pharmazeutisch verträgliche Lösungsmittel können erfindungsgemäß alle Lösungsmittel in Betracht gezogen werden, die pharmazeutisch venträglich sind und nicht mit dem Wirkstoff sowie eventuell weiteren Inhaltsstoffen des Medikaments bzw. der pharmazeutischen Lösungsformulierung unverträglich sind. Erfindungsgemäß sind besonders geeignete pharmazeutisch verträgliche Lösungsmittel Wasser, insbesondere Wasser für Injektionszwecke, sowie Wasser umfassend Isotonisierungszusätze und Kochsalzlösung, insbesondere isotonische Kochsalzlösung. Am meisten bevorzugt ist Wasser für Injektionszwecke. Als beispielhafte Isotonisierungszusätze können lösliche Salze (Natriumchlorid, Kaliumchlorid), Zucker (Glucose, Lactose), Zuckeralkohole (Mannitol, Sorbitol) sowie Kombinationen aus diesen Hilfsstoffen erfindungsgemäß verwendet werden.

Neben Isotonierungszusätzen kann das erfindungsgemäße Medikament ferner im Fachgebiet der pharmazeutischen Lösungsformulierungen übliche Zusatzstoffe beinhalten. Insbesondere kann das erfindungsgemäße Medikament übliche Hilfsstoffe mit folgender Funktionalität enthalten: Eu-/ Isohydrisierung (Acetat-, Phosphat-, Citratpuffer), Antioxidantien (Ascorbinsäure, im Fachgebiet übliche Schwefelverbindungen), Lösungsvermittlung (Komplexbildner, Solubilisatoren, Cosolventien: z.B. Cyclodextrine, Polyvidon, Polysorbate, Lecithin, Glykocholat), Viskositätserhöhung, pH-Einstellung (Säuren, Basen, bzw. saure oder basische Salze). In einer besonders bevorzugten Ausführungsform liegt der pH-Wert des erfindungsgemäßen Medikaments zwischen 7,5 und 9.

Die erfindungsgcmäßen Medikamente sind auf die Behandlung von entzündlichen Autoimmunerkrankungen gerichtet. Der Begriff "entzündliche Autoimmunerkrankung" umfasst sämtliche entzündlichen Autoimmunerkrankungen, die mit Methotrexat sinnvoll therapiert werden können. Als nicht einschränkende Beispiele für entzündliche Autoimmunerkrankungen, welche mit den erfindungsgemäßen Medikamenten behandelt werden können, können rheumatoide Arthritis, juvenile Arthritiden, Vaskulitiden, Kollagenosen, Morbus Crohn, Colitis Ulcerosa, Asthma bronchiale, Morbus Alzheimer, Multiple Sklerose, Morbus Bechterew, Gelenkarthrosen oder Psoriasis, sowie Psoriasis Arthritis und insbesondere Psoriasis vulgaris vom Plaque-Typ genannt werden. Besonders bevorzugt eignen sich die erfindungsgemäßen Medikamente zur Behandlung von rheumatoider Arthritis, einschließlich juveniler Arthritiden, wie speziell die olisoarthritische und polyarthritische Form der juvenilen Arthritis.

Die Verabreichung der erfindungsgemäßen Medikamente erfolgt parenteral. Insbesondere erfolgt die Applikation der Medikamente intravenös, intramuskulär oder subkutan durch Injektion. Gemäß einer bevorzugten Ausführungsform der vorliegenden Erfindung liegt das Medikament in einer derartigen Form vor, dass es zu einer subkutanen Verabreichung geeignet ist. Weiterhin ist es bevorzugt, dass das Medikament in einer Form vorliegt, die es ermöglicht, dass die subkutane Verabreichung des Medikaments durch den Patienten selbst vorgenommen wird (Selbstapplikation). Eine derartige Therapie der subkutanen Selbstapplikation hat sich beispielsweise bei der Verabreichung von Insulin durch den betroffenen Diabetiker selber bewährt und führt zu einer hohen Therapie-Akzeptanz durch den Patienten ("Patienten-Compliance"). Im Falle der Rheumatherapie kann bei Selbstapplikation zudem auf den in der Regel wöchentlich zu erfolgenden Arztbesuch verzichtet werden.

In einer bevorzugten Ausführungsform der vorliegenden Erfindung ist das erfindungsgemäße Medikament in einer Injektionsvorrichtung zur Einfachapplikation, insbesondere einer Fertigspritze, enthalten. Unter einer Injektionsvorrichtung zur Einfachapplikation ist erfindungsgemäß eine Vorrichtung zu verstehen, welche neben einem Behältnis, welches die erfindungsgemäße pharmazeutische Lösungsformulierung beinhaltet, eine Injektionsnadel (Kanüle) umfasst, wobei durch diese das Medikament dem Patienten appliziert werden kann. Ferner umfasst eine derartige Injektionsvorrichtung eine mechanische Anordnung (z.B. einen Stempel oder eine flexible Blase), mit deren Hilfe das Medikament durch die Injektionsnadel aus dem Behältnis gedrückt werden kann. Eine derartige Injektionsvorrichtung zur Einfachapplikation ist ferner **dadurch gekennzeichnet, dass** sie eine gewünschte Einzeldosierung des Wirkstoffs enthält und somit bei der Applikation das Behältnis, welches die erfindungsgemäße pharmazeutische Lösungsformulierung beinhaltet, vollständig zu entleeren ist, um die vorgesehene Dosierung zu verabreichen. Aufgrund dieser Tatsache kann gemäß der vorstehenden Ausführungsform in der Regel auf die Beimischung eines Konservierungsmittels zu der pharmazeutischen Lösungsformulierung von Methotrexat verzichtet werden.

In einer erfindungsgemäßen Injektionsvorrichtung zur Einfachapplikation ist vorzugsweise eine Dosis des Wirkstoffs Methotrexat von 5mg bis 40mg enthalten. Besonders bevorzugt enthält eine Injektionsvorrichtung zur Einfachapplikation gemäß der Erfindung eine Dosis von 5,0, 7,5, 10,0, 12,5, 15,0, 17,5, 20,0, 22,5, 25,0, 27,5, 30,0, 32,5, 35,0, 37,5 oder 40,0mg. Das zur Bereitstellung einer jeweils gewünschten Dosierung erforderliche FlüssigkeitsVolumen, welches in der Injektionsvorrichtung zur Einfachapplikation enthalten sein muss, hängt von der jeweils gewählten Konzentration der Wirkstofflösung ab und erscbließt sich dem Fachmann in einfacher Weise. Demnach müsste zur Bereitstellung einer Wirkstoffdosis von 30.0mg bei einer Konzentration an Methotrexat in dem pharmazeutisch verträglichen Lösungsmittel von beispielsweise 50mg/ml eine Injektionsvorrichtung zur Einfachapplikation ein Flüssigkeitsvolumen von 0,6ml enthalten.

Ein besonders bevorzugtes Beispiel für eine erfindungsgemäße Injektionsvorrichtung zur Einfachapplikation ist eine Fertigspritze. Fertigspritze sind auf dem pharmazeutischen Fachgebiet gut bekannt, insbesondere auch auf dem Gebiet der Therapie der rheumatoiden Arthritis mit Methotrexat. Auf dem deutschen Markt werden bereits Fertigspritzen enthaltend Methotrexat-Lösungen mit Konzentrationen von 7,5mg/ml, 10,0mg/ml und 25mg/ml vertrieben (Handelsnamen: Lantarel® der Firma Wyeth, Metex® der Anmelderin, wobei das Handelsprodukt Lantarel® FS 25 mg nicht zur subkutanen Applikation zugelassen ist). Obwohl die Bereitstellung von Methotrexat-Lösungen in Fertigspritzen, mitunter zur Selbstapplikation, die Patienten-Compliance positiv beeinflusst, haben die zur subkutanen Applikation zugelassenen Präparate des Standes der Technik den Nachteil, dass, abhängig von der wöchentlich zu verabreichenden Wirkstoffmenge, verhältnismäßig große Flüssigkeitsmengen unter die Haut des Patienten gebracht werden müssen. Bei einer üblichen wöchentlichen Wirkstoffdosis von 30mg bedeutet dies, dass bei der derzeit höchstkonzentrierten Wirkstofflösung des Standes der Technik zur subkutanen Applikation, nämlich 10mg/ml (im Handelsprodukt Metex® 10mg/ml der Arnnelderin), ein Volumen von 3ml unter die Haut gespritzt werden muss. Diese hohe Flüssigkeitsmenge ist dem Patienten, insbesondere Kindern, mitunter schwer vermittelbar, was zu einer verminderten Patienten-Compliance führt.

Die erfindungsgemäß bereitgestellten Medikamente enthalten dagegen hochkonzentrierte Lösungen des Wirkstoffs Methotrexat, wodurch sich die zu verabreichende Flüssigkeitsmenge bei einer bestimmten wöchentlichen Wirkstoffdosis verringert. Beispielsweise wäre es demnach bei einer erfindungsgemäß besonders bevorzugten Konzentration von 50mg/ml zur Einhaltung einer wöchentlichen Wirkstoffdosis von 30mg ausreichend, ein Flüssigkeitsvolumen von lediglich 0.6ml subkutan zu verabreichen. Es kann erwartet werden, dass dies positive Auswirkungen auf die Patienten-Compliance hat.

In einer bevorzugten Ausführungsform stellt die vorliegende Erfindung demnach eine Fertigspritze bereit, enthaltend eine pharmazeutische Lösungsformulierung von Methotrexat mit einer Konzentration von größer als 25mg/ml in einem pharmazeutisch verträglichen Lösungsmittel. Fertigspritze sind auf dem pharmazeutischen Fachgebiet gut bekannt und sind erfindungsgemäß nicht besonders eingeschränkt. Erfindungsgemäße Fertigspritzen umfassend beispielsweise auch Einweg-Injektionssysteme, wie das Uniject®-Injektionssystem. In einer Ausführungsform kann die Fertigspritze bereits mit einer geeigneten Kanüle zur intravenösen, intramuskulären oder subkutanen Injektion versehen sein, in einer alternativen Ausführungsform ist die Fertigspritze zunächst mit einer Gummikappe oder dgl. versehen, die vor der Applikation durch den Arzt, das medizinische Personal oder, bei subkutaner Selbstapplikation, durch den Patienten selber durch eine separat steril verpackte Kanüle ersetzt wird.

Vorzugsweise ist die erfindungsgemäße Fertigspritze so ausgestaltet, dass sie zur subkutanen Applikation der Wirkstofflösung geeignet ist, was insbesondere durch Bereitstellung einer zur Subkutaninjektion geeigneten Kanüle bewerkstelligt werden kann. In einer bevorzugteren Ausführungsform ist die Fertigspritze konstruktiv so ausgestaltet, dass auch Rheunlapatienten, die eine eingeschränkte manuelle Feinmotorik aufweisen und daher nicht ausnahmslos in der Lage sind, sich selbst ein Medikament mit herkömmlich ausgestaltete Fertigspritzen zu injizieren, eine Selbstapplikation durchführen können. Hierzu sind vorzugsweise insbesondere Stempel und back stopp derart konstruiert und dimensioniert, so dass dem Rheumapatienten die Handhabung erleichtert ist. Derartig ausgestaltete Fertigspritzen sind auf dem Fachgebiet bekannt.

In einer weiteren bevorzugten Ausführungsform, der vorliegenden Erfindung ist das erfindungsgemäße Medikament in einem Vorratsgefäß enthalten. Unter einem Vorratsgefäß ist erfindungsgemäß jedes auf dem Fachgebiet übliche Behältnis zu verstehen, in welches/welchem das Medikament bzw. die pharmazeutische Lösungsformulierung der Erfindung fachgerecht, d.h. insbesondere steril, abgefüllt und aufbewahrt werden kann. Als nicht beschränkende Beispiele für Vorratsgefäße im Sinne der Erfindung können eine Durchstechflasche, ein Vial, ein Beutel, eine Glasampulle oder eine Karpule genannt werden. Gemäß einer Ausführungsform der Erfindung muss zur Applikation des Medikaments an den Patienten zunächst die gewünschte Menge an pharmazeutischer Lösungsformulierung mittels einer Injektionsvorrichtung (beispielsweise eine herkömmliche Einwegspritze) aus dem Vorratsgefäß (beispielsweise eine Durchstechflasche) entnommen werden, während gemäß einer alternativen Ausführungsform der Erfindung die pharmazeutische Lösungsformulierung mittels einer Injektionsvorrichtung (beispielsweise ein Pen-Injektor) direkt aus dem Vorratsgefäß (beispielsweise eine Karpule) appliziert werden kann.

In einer bevorzugten Ausführungsform der Erfindung umfasst das Vorratsgefäß neben dem Wirkstoff Methotrexat, welcher in dem pharmazeutisch verträglichen Lösungsmittel gelöst ist, zumindest noch ein Konservierungsmittel. Das erfindungsgemäß verwendbare Konservierungsmittel ist nicht besonders eingeschränkt und ein Fachmann wird ohne Schwierigkeiten in der Lage sein, aus den bekannten Konservierungsmitteln für pharmazeutische Zwecke ein geeignetes auszuwählen. Als bevorzugte Konservierungsmittel können. Cresole, Benzylalkohole, Phenylethylalkohole genannt werden. Das Konservierungsmittel dient insbesondere dazu, die bei einer Teilentnahme des Medikaments (beispielsweise durch eine herkömmliche Einwegspritze oder einen Pen-Injektor) in einem erfindungsgemäßen Vorratsgefäß (beispielsweise eine Durchstechflasche oder eine Karpule) verbleibende pharmazeutische Losungsformulierung zu konservieren.

Die Gesamtdosienmgsmenge an dem Wirkstoff Methotrexat in einem erfindungsgemäßen Vorratsgefäß ist nicht besonders begrenzt und wird neben der verwendeten Konzentration von Methotrexat in dem pharmazeutisch verträglichen Lösungsmittel insbesondere durch die Abmessungen des Vorratsgefäßes und somit vom dem Flüssigkeitsvolumen bestimmt, welches das Vorratsgefäß aufnehmen kann. Bevorzugt enthält ein erfindungsgemäßes Vorratsgefäß eine Gesamtdosierungsmenge von 5 bis 5000 mg Methotrexat.

Als bevorzugtes Beispiel für ein Vorratsgefäß, in welchem das erfindungsgemäße Medikament enthalten ist, kann eine Karpule genannt werden. Karpulen, auch Zylinderampullen genannt, sind auf dem Fachgebiet gut bekannt. Unter einer Karpule versteht der Fachmann einen vorzugsweise zylinderförmigen, sterilen Medikanientenbehälter, welcher vorzugsweise aus Glas oder einem möglichst durchsichtigen, inerten Kunststoff (z.B.: Topas®) gefertigt ist. Auf der einen Seite des Karputenzylinders befindet sich üblicherweise ein beweglicher Endstopfen, auf der gegenüberliegenden Seite eine durchstechbare Membran aus Gummi oder einem vergleichbaren elastischen Verschlussstoff. Zur Applikation wird das in der Karpule befindliche pharmazeutische Präparat durch Einwirken z.B. eines externes Stempels oder Kolbens auf den beweglichen Endstopfen durch eine Kanüle, welche die beschriebene Gummimembran durchsticht, aus der Karpule gepresst.

In einer weiteren Ausführungsform stellt die vorliegende Erfindung demnach eine Karpule bereit, enthaltend eine pharmazeutische Lösungsformulierung von Methotrexat mit einer Konzentration von größer als 25mg/ml in einem pharmazeutisch verträglichen Lösungsmittel. In einer bevorzugten Ausführungsform enthält eine erfindungsgemäße Karpule eine Gesamtdosierungsmenge von 5 bis 500mg, besonders bevorzugt 7,5 bis 300mg, Methotrexat.

Vorzugsweise wird die Verabreichung des Medikaments aus der Karpule mittels einer Injektionsvorrichtung vorgenommen. In einer besonders bevorzugten Ausführungsform der Erfindung ist demnach die Karpule zur Verabreichung des Medikaments mittels einer Injektionsvorrichtung geeignet. Derartige Injektionsvorrichtungen sind auf dem Fachgebiet gut bekannt. Als eine solche Injektionsvorrichtung ist vorzugsweise ein sogenannter Pen-Injektor ("Pen") zu nennen, in welchen die Karpule eingesetzt werden kann. Pen-Injektoren sehen in der Regel aus wie größere Füllfederhalter (auf englisch "pen") und sind insbesondere Diabetikern geläufig, die sich mit deren Hilfe bequem die notwendige Dosis Insulin verabreichen können. Nach erfolgter Entleerung der eingesetzten Karpule kann in den Pen-Injektor auf einfache Weise eine neue Karpule eingesetzt werden (vergleichbar dem Austausch einer Tintenpatrone in dem zuvor zum Vergleich genannten Füllfederhalter).

In einer weiteren Ausführungsform stellt die vorliegende Erfindung demnach einen Pen-Injektor bereit, umfassend eine vorstehend beschriebene, erfindungsgemäße Karpule, die das erfindungsgemäße Medikament enthält.

Vorzugsweise ist ein erfindungsgemäßer Pen-Injektor so ausgestaltet, dass er zur subkutanen Applikation der Wirkstofflösung geeignet ist, was insbesondere durch Bereitstellung einer zur Subkutaninjektion geeigneten Kanüle bewerkstelligt werden kann. Ferner ist ein erfindungsgemäßer Pen-Injektor sowie die darin umfasste Karpule vorzugsweise derart ausgestaltet, dass eine Mehrfachapplikation von Einzeldosierungen erfolgen kann. Hierzu ist ein erfindungsgemäßer Pen-Injektor vorzugsweise mit einer konstruktiven Vorrichtung versehen (z.B. einem Regelrad), mit deren Hilfe die Einstellung einer bestimmten Dosierung (d.h. konkret die Auswahl eines bestimmten Applikationsvolumens bei bekannter Wirkstoffkonzentration von Methotrexat in der pharmazeutischen Lösungsformulierung) des zu verabreichenden Methotrexats durch den Arzt, das medizinische Personal, oder, bei Selbstapplikation, durch den Patienten selbst vorgenommen werden kann. Somit stellt die Erfindung mit dieser Ausführungsform die Möglichkeit bereit, bei Bedarf auch Zwischendosicrungen auszuwählen, für die keine sonstigen Vorratsgefäße oder Injektionsvorrichtungen, insbesondere keine Durchstechflaschen oder Fertigspritzen, kommerziell erhältlich sind. Derartig konstruierte Pen-Injektoren sind auf dem Fachgebiet, insbesondere aus dem Insulinbereich, gut bekannt.

Gemäß einer besonders bevorzugten Ausführungsform der Erfindung ist ein erfindungsgemäßer Pen-Injektor derart ausgestaltet, dass die Einzeldosierungen pro Applikation auf jeweils von 5 bis 40mg Methotrexat eingestellt werden kann. Insbesondere kann ein erfindungsgemäßer Pen-Injektor derart eingestellt werden, dass pro Applikation jeweils eine Einzeldosis von 5,0, 7,5, 10,0, 12,5, 15,0, 17,5, 20,0, 22,5, 25,0, 27,5, 30,0, 32,5, 35,0, 37,5 oder 40,0mg verabreicht werden kann.

Die Anmeldung wird durch folgende Punkte zusammengefasst:
1. Verwendung von Methotrexat zur Herstellung eines parenteral zu verabreichenden Medikaments zur Behandlung von entzündlichen Autoimmunerkrankungen, wobei das Methotrexat in einer Konzentration von größer als 25 mg/ml in einem pharmazeutisch verträglichen Lösungsmittel vorliegt.
2. Verwendung nach Punkt 1, wobei das Methotrexat in einer Konzentration von größer als 25 mg/ml bis 150 mg/ml vorliegt.
3. Verwendung nach Punkt 2, wobei das Methotrexat in einer Konzentration von etwa 50 mg/ml vorliegt.
4. Verwendung nach einem der vorangehenden Punkte, wobei das pharmazeutisch verträgliche Lösungsmittel aus Wasser, Wasser für Injektionszwecke, Wasser umfassend Isotonisierungszusätze und Kochsalzlösung, insbesondere isotonische Kochsalzlösung, ausgewählt ist.
5. Verwendung nach einem der vorangehenden Punkte, wobei die entzündliche Autoimmunerkrankung ausgewählt ist aus rheumatoide Arthritis, juvenile Arthritiden, Vaskulitiden, Kollagenosen, Morbus Crohn, Colitis Ulcerosa, Astma bronchiale, Morbus Alzheimer, Multiple Sklerose, Morbus Bechterew, Gelenkarthrosen oder Psoriasis.
6. Verwendung nach Punkt 5, wobei die entzündliche Autoimmunerkrankung rheumatoide Arthritis, insbesondere juvenile rheumatoiden Arthritis, ist.
7. Verwendung nach einem der vorangehenden Punkte, wobei das Medikament in einer zur subkutanen Verabreichung geeigneten Form vorliegt.
8. Verwendung nach Punkt 7, wobei das Medikament in einer zur Verabreichung durch den Patienten selbst geeigneten Form vorliegt.
9. Verwendung nach einem der vorangehenden Punkte, wobei das Medikament in einer Injektionsvorrichtung zur Einfachapplikation enthalten ist.
10. Verwendung nach Punkt 9 wobei die Injektionsvorrichtung eine Dosierung von 5 bis 40 mg, insbesondere 5,0, 7,5, 10,0, 12,5, 15,0, 17,5, 20,0, 22,5, 25,0, 27,5, 30,0, 32,5, 35,0, 37,5 oder 40,0 mg Methotrexat enthält.
11. Verwendung nach Punkt 9 oder 10, wobei die Injektionsvorrichtung eine Fertigspritze ist.
12. Verwendung nach einem der Punkte 1 bis 8, wobei das Medikament in einem Vorratsgefäß enthalten ist.
13. Verwendung nach Punkt 12, wobei das Medikament ferner ein Konservierungsmittel umfasst.
14. Verwendung nach Punkt 12 oder 13, wobei das Vorratsgefäß eine Gesamtdosierungsmenge von 5 bis 5000 mg enthält.
15. Verwendung nach einem der Punkte 12 bis 14, wobei das Vorratsgefäß eine Durchstechflasche, ein Vial, ein Beutel, eine Glasampulle oder eine Karpule ist.
16. Verwendung nach Punkt 15, wobei das Vorratsgefäß eine Karpule ist und diese zur Verabreichung des Medikaments mittels einer Injektionsvorrichtung, insbesondere eines Pen-Injektors, geeignet ist.
17. Verwendung nach Punkt 16, wobei die Karpule und der Pen-Injektor derart ausgestaltet sind, dass eine Mehrfachapplikation von Einzeldosierungen erfolgen kann.
18. Verwendung nach Punkt 17, wobei die Einzeldosierungen pro Applikation auf jeweils von 5 bis 40 mg, insbesondere jeweils 5,0, 7,5, 10,0, 12,5, 15,0, 17,5, 20,0, 22,5, 25,0, 27,5, 30,0, 32,5, 35,0, 37,5 oder 40,0 mg Methotrexat eingestellt werden können.
19. Fertigspritze, enthaltend eine pharmazeutische Lösungsformulierung von Methotrexat mit einer Konzentration von größer als 25 mg/ml in einem pharmazeutisch verträglichen Lösungsmittel.
20. Fertigspritze nach Punkt 19, wobei das Methotrexat in einer Konzentration von größer als 25 mg/ml bis 150 mg/ml vorliegt.
21. Fertigspritze nach Punkt 20, wobei das Methotrexat in einer Konzentration von etwa 50 mg/ml vorliegt.
22. Fertigspritze nach einem der Punkte 19 bis 21, wobei diese eine Dosierung von 5 bis 40 mg, insbesondere 5,0, 7,5, 10,0, 12,5, 15,0, 17,5, 20,0, 22,5, 25,0, 27,5, 30,0, 32,5, 35,0, 37,5 oder 40,0 mg Methotrexat enthält.
23. Fertigspritze nach einem der Punkte 19 bis 22, wobei das pharmazeutisch verträgliche Lösungsmittel aus Wasser, Wasser für Injektionszwecke, Wasser umfassend Isotonisierungszusätze und Kochsalzlösung, insbesondere isotonische Kochsalzlösung, ausgewählt ist.
24. Fertigspritze nach einem der Punkte 19 bis 23 zur subkutanen Verabreichung.
25. Fertigspritze nach Punkt 24, wobei die Fertigspritze konstruktiv so ausgestaltet ist, dass einem Patienten mit eingeschränkter Feinmotorik die Selbstapplikation ermöglicht wird.
26. Karpule, enthaltend eine pharmazeutische Lösungsformulierung von Methotrexat mit einer Konzentration von größer als 25 mg/ml in einem pharmazeutisch verträglichen Lösungsmittel.
27. Karpule nach Punkt 26, wobei das Methotrexat in einer Konzentration von größer als 25 mg/ml bis 150 mg/ml vorliegt.
28. Karpule nach Punkt 27, wobei das Methotrexat in einer Konzentration von etwa 50 mg/ml vorliegt.
29. Karpule nach einem der Punkte 26 bis 28, wobei die pharmazeutische Lösungsformulierung ferner ein Konservierungsmittel enthält.
30. Karpule nach einem der Punkte 26 bis 29, wobei diese eine Gesamtdosierungsmenge von 5 bis 500 mg, insbesondere 7,5 bis 300 mg, Methotrexat enthält.
31. Karpule nach einem der Punkte 26 bis 30, wobei das pharmazeutisch verträgliche Lösungsmittel aus Wasser, Wasser für Injektionszwecke, Wasser umfassend Isotonisierungszusätze und Kochsalzlösung, insbesondere isotonische Kochsalzlösung, ausgewählt ist.
32. Pen-Injektor, umfassend eine Karpule gemäß einem der Punkte 26 bis 31.
33. Pen-Injektor nach Punkt 32 zur subkutanen Verabreichung.
34. Pen-Injektor nach Punkt 32 oder 33, wobei die Karpule und der Pen-Injektor derart ausgestaltet sind, dass eine Mehrfachapplikation von Einzeldosierungen erfolgen kann.
35. Pen-Injektor nach Punkt 34, wobei die Einzeldosierungen pro Applikation auf jeweils von 5 bis 40 mg, insbesondere jeweils 5,0, 7,5, 10,0, 12,5, 15,0, 17,5, 20,0, 22,5, 25,0, 27,5, 30,0, 32,5, 35,0, 37,5 oder 40,0 mg Methotrexat eingestellt werden können.

Die nachfolgenden Beispiele verdeutlichen die Erfindung, ohne sie zu beschränken:

### Beispiele

### Beispiel 1:

Die nachfolgend veranschaulichte Methotrexat-Lösung (Konzentration: 50 mg/ml) wurde unter Zugrundelegung folgender Rezepturmengen hergestellt.

| | | |
|---|---|---|
| Methotrexat: | 1.500g | |
| Natriumchlorid: | 120g | |
| Natriumhydroxid: | 300g | |
| Wasser für Injektionszwecke: | 28.764g | |
| Gesamt: | 30.684g | = 30 liter |

Zur Herstellung der Lösung (Beispiel 1) wurden etwa 60% des benötigten Wassers für Injektionszwecke (20-25°C) im Ansatzbehälter vorgelegt. Unter eingeschaltetem Rührwerk wurde die angegebene Menge an Natriumchlorid hinzugegeben und vollständig gelöst, Der Behälter und die Lösung wurde mit Stickstoff geflutet wodurch der Restsauerstoff weitgehend verdrängt wurde. Die angegebene Menge an Methotrexat wurde in der Lösung unter weiter eingeschaltetem Rührwerk suspendiert. Der pH-Wert der Lösung wurde unter Verwendung von 1%iger Natronlauge (Hergestellt aus NaOH und Wasser für Injektionszwecke) auf einen Wert zwischen 8,5 bis 8,9 eingestellt. Die Temperatur der Lösung liegt hierbei zwischen 20 bis 30°C. Es entsteht eine klare Lösung, deren pH-Wert stabil zwischen 8,5 und 8,9 liegt. Durch Zusatz von der Restmenge Wasser für Injektionszwecke wurde auf das endgültige Volumen aufgefüllt.

Durch Sterilfiltration über einen 0,22 µm Sterilfilter wurde die Lösung unter Verwendung von Schutzgas (Stickstoff) in die vorgesehenen sterilen Glasbehälter der Glasart 1 (Karpulen oder Fertigspritzen) unter Reinraumbedingungen (Klasse A) gefüllt.

### Beispiel 2:

Die nachfolgend veranschaulichte Methotrexat-Lösung (Konzentration: 50 mg/ml) wurde unter Zugrundelegung folgender Rezepturmengen hergestellt.

| | | |
|---|---|---|
| Methotrexatdinatrium: | 1.645g | |
| Natriumchlorid: | 120g | |
| Wasser für Injektionszwecke: ad 30.684g | | |
| Gesamt: | 30.684g | = 30 liter |

Zur Herstellung der Lösung (Beispiel 2) wurden etwa 60% des benötigten Wassers für Injektionszwecke (20-25°C) im Ansatzbehälter vorgelegt. Unter eingeschaltetem Rührwerk wurde die angegebene Menge an Natriumchlorid hinzugegeben und vollständig gelöst. Der Behälter und die Lösung wurde mit Stickstoff geflutet wodurch der Restsauerstoff weitgehend verdrängt wurde. Die angegebene Menge an Methotrexatdinatriumsalz wurde in der Lösung unter weiter eingeschaltetem Rührwerk gelöst. Die Temperatur der Lösung liegt hierbei zwischen 20 bis 30°C. Die Lösung ist klar und der pH-Wert liegt stabil zwischen 8,5 und 8,9. Durch Zusatz von der Restmenge Wasser für Injektionszwecke wurde auf das endgültige Volumen aufgefüllt.

Durch Sterilfiltration über einen 0,22 µm Sterilfilter wurde die Lösung unter Verwendung von Schutzgas (Stickstoff) in die vorgesehenen sterilen Glasbehälter der Glasart 1 (Karpulen oder Fertigspritzen) unter Reinraumbedingungen (Klasse A) gefüllt.

## Patentansprüche

1. Verwendung von Methotrexat zur Herstellung eines subkutan zu verabreichenden Medikaments zur Behandlung von entzündlichen Autoimmunerkrankungen, wobei das Methotrexat in einer Konzentration von größer als 25 mg/ml in einem pharmazeutisch verträglichen Lösungsmittel vorliegt.

2. Verwendung nach Anspruch 1, wobei das Methotrexat in einer Konzentration von größer als 25 mg/ml bis 150 mg/ml vorliegt.

3. Verwendung nach Anspruch 1, wobei das Methotrexat in einer Konzentration von 30 mg/ml bis 100 mg/ml, vorzugsweise 40 mg/ml bis 80 mg/ml, insbesondere vorzugsweise 50 mg/ml bis 75 mg/ml vorliegt.

4. Verwendung nach Anspruch 3, wobei das Methotrexat in einer Konzentration von größer etwa 50 mg/ml vorliegt.

5. Verwendung nach einem der vorangehenden Ansprüche, wobei das pharmazeutisch verträgliche Lösungsmittel aus Wasser, Wasser für Injektionszwecke, Wasser umfassend Isotonisierungszusätze und Kochsalzlösung, insbesondere isotonische Kochsalzlösung, ausgewählt ist.

6. Verwendung nach einem der vorangehenden Ansprüche, wobei die entzündliche Autoimmunerkrankung ausgewählt ist aus rheumatoide Arthritis, juvenile Arthritiden, Vaskulitiden, Kollagenosen, Morbus Crohn, Colitis Ulcerosa, Astma bronchiale, Morbus Alzheimer, Multiple Sklerose, Morbus Bechterew, Gelenkarthrosen oder Psoriasis.

7. Verwendung nach Anspruch 6, wobei die entzündliche Autoimmunerkrankung rheumatoide Arthritis, insbesondere juvenile rheumatoide Arthritis, ist.

8. Verwendung nach einem der vorangehenden Ansprüche, wobei das Medikament in einer zur Verabreichung durch den Patienten selbst geeigneten Form vorliegt.

9. Verwendung nach einem der vorangehenden Ansprüche, wobei das Medikament in einer Injektionsvorrichtung zur Einfachapplikation enthalten ist.

10. Verwendung nach Anspruch 9, wobei die Injektionsvorrichtung eine Fertigspritze ist.

11. Verwendung nach einem der Ansprüche 1 bis 8, wobei das Medikament in einem Vorratsgefäß enthalten ist.

12. Verwendung nach Anspruch 11, wobei das Medikament ferner ein Konservierungsmittel umfasst.

13. Verwendung nach einem der Ansprüche 11 oder 12, wobei das Vorratsgefäß eine Durchstechflasche, ein Vial, ein Beutel, eine Glasampulle oder eine Karpule ist.

14. Verwendung nach Anspruch 13, wobei das Vorratsgefäß eine Karpule ist und diese zur Verabreichung des Medikaments mittels einer Injektionsvorrichtung, insbesondere eines Pen-Injektors, geeignet ist.

15. Verwendung nach Anspruch 14, wobei die Karpule und der Pen-Injektor derart ausgestaltet sind, dass eine Mehrfachapplikation von Einzeldosierungen erfolgen kann.

16. Verwendung nach Anspruch 10, wobei die Fertigspritze eine Dosierung von 5 bis 40 mg, insbesondere 5,0, 7,5, 10,0, 12,5, 15,0, 17,5, 20,0, 22,5, 25,0, 27,5, 30,0, 32,5, 35,0, 37,5 oder 40,0 mg Methotrexat enthält.

17. Verwendung nach einem der Ansprüche 10 oder 16, wobei die Fertigspritze konstruktiv so ausgestaltet ist, dass einem Patienten mit eingeschränkter Feinmotorik die Selbstapplikation ermöglicht wird.
